(19) Europäisches Patentamt

European Patent Office

Office européen des brevets

(11) EP 1 736 101 A1

## (12) EUROPEAN PATENT APPLICATION

(43) Date of publication:
27.12.2006 Bulletin 2006/52

(51) Int Cl.:
A61B 6/03 (2006.01)
G01N 23/04 (2006.01)
G06T 11/00 (2006.01)

(21) Application number: 06253221.3

(22) Date of filing: 22.06.2006

(84) Designated Contracting States:
AT BE BG CH CY CZ DE DK EE ES FI FR GB GR
HU IE IS IT LI LT LU LV MC NL PL PT RO SE SI
SK TR
Designated Extension States:
AL BA HR MK YU

(30) Priority: 24.06.2005 JP 2005184722

(71) Applicant: GE Medical Systems Global
Technology Company, LLC
Waukesha,
Wisconsin 53188-1696 (US)

(72) Inventors:
• Nishide, Akihiko
  Hino-shi,
  Tokyo 191-8503 (JP)
• Hagiwara, Akira
  Hino-shi,
  Tokyo 191-8503 (JP)
• Imai, Yasuhiro
  Hino-shi,
  Tokyo 191-8503 (JP)

(74) Representative: Pedder, James Cuthbert
London Patent Operation
General Electric International Inc.
15 John Adam Street
GB-London WC2N 6LU (GB)

(54) **Computer axial tomograph utilizing a two-dimensional detector and a biological synchronization signal**

(57) When an X-ray CT apparatus (100) including a two-dimensional X-ray area detector (24) performs a conventional (axial) scan or a cine scan, a plurality of segments of projection data items detected synchronously with an external signal or a biomedical signal is sampled from projection data items acquired during one scan or a plurality of scans. Projection data items corresponding to those detected during a half scan of rotating an X-ray tube (21) by an angle of 180° plus the angle of a fan beam falling on the detector (24) or during a 360° full scan are used to reconstruct a three-dimensional image. Thus, a three-dimensional tomographic image enjoying a high temporal resolution is displayed. Moreover, successive three-dimensional display images showing states of a subject synchronized with respective phases of the external signal or biomedical signal may be displayed as a four-dimensional image.

Fig. 1

EP 1 736 101 A1

**Description**

The present invention relates to an X-ray computed tomography (CT) method to be implemented in X-ray CT apparatuses for medical or industrial use, and an X-ray CT apparatus to be adapted to the X-ray CT apparatuses for medical or industrial use. More particularly, the present invention relates to conventional (axial) or cine scan synchronous imaging, three-dimensional display, and four-dimensional display or to improvement of image quality.

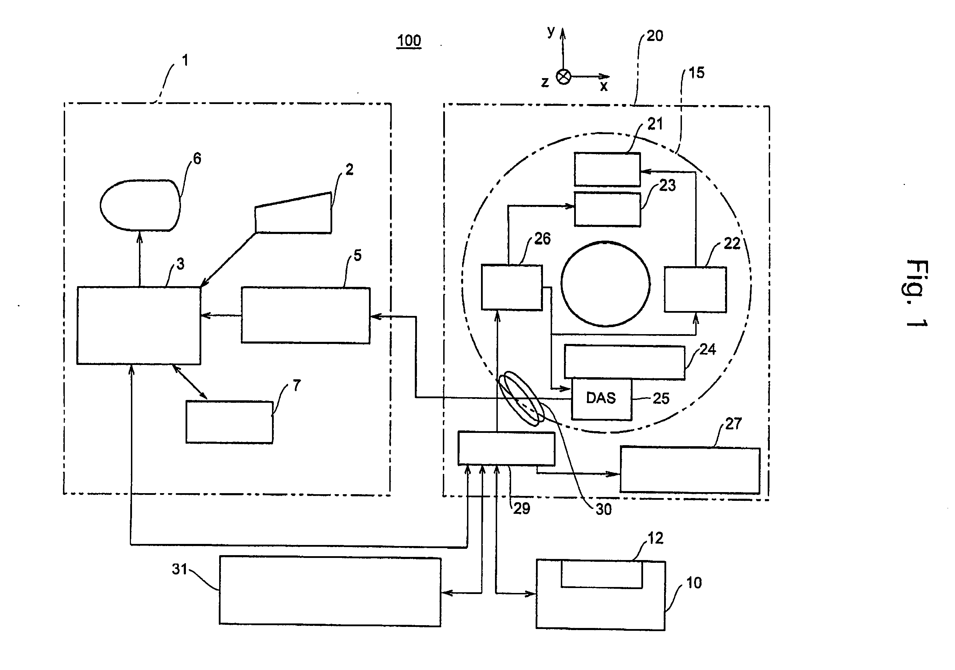

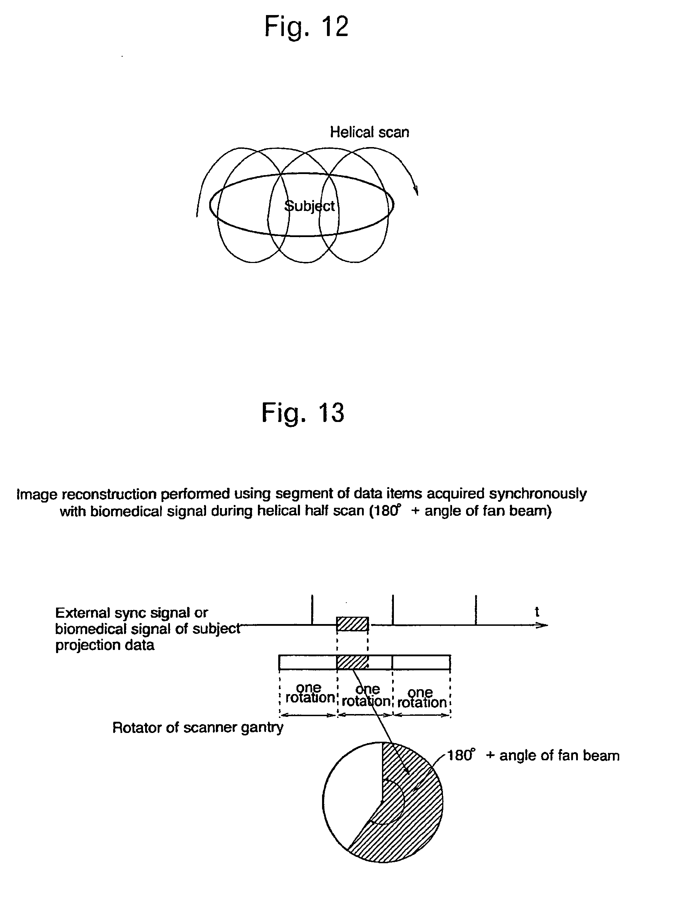

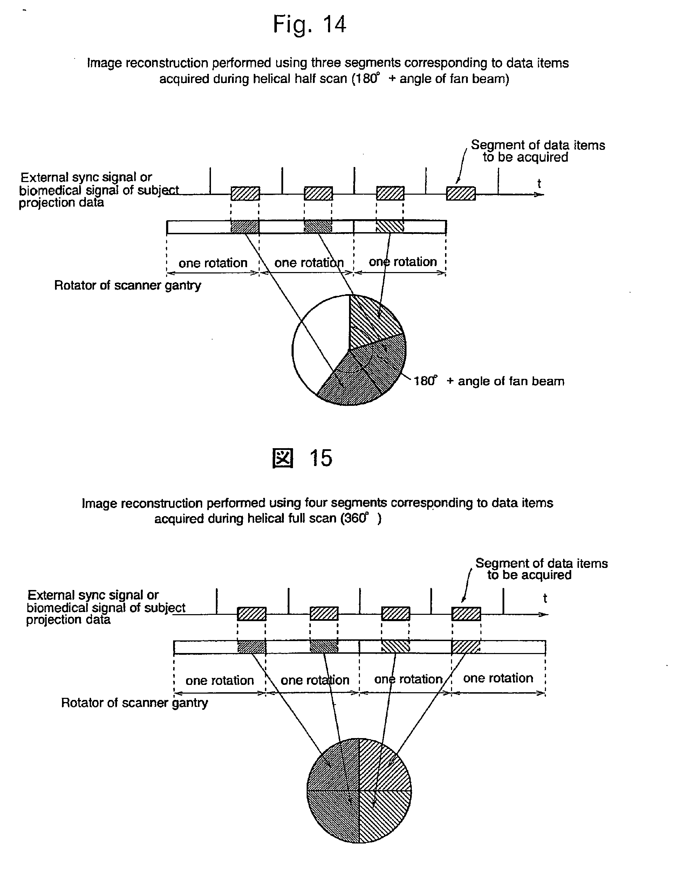

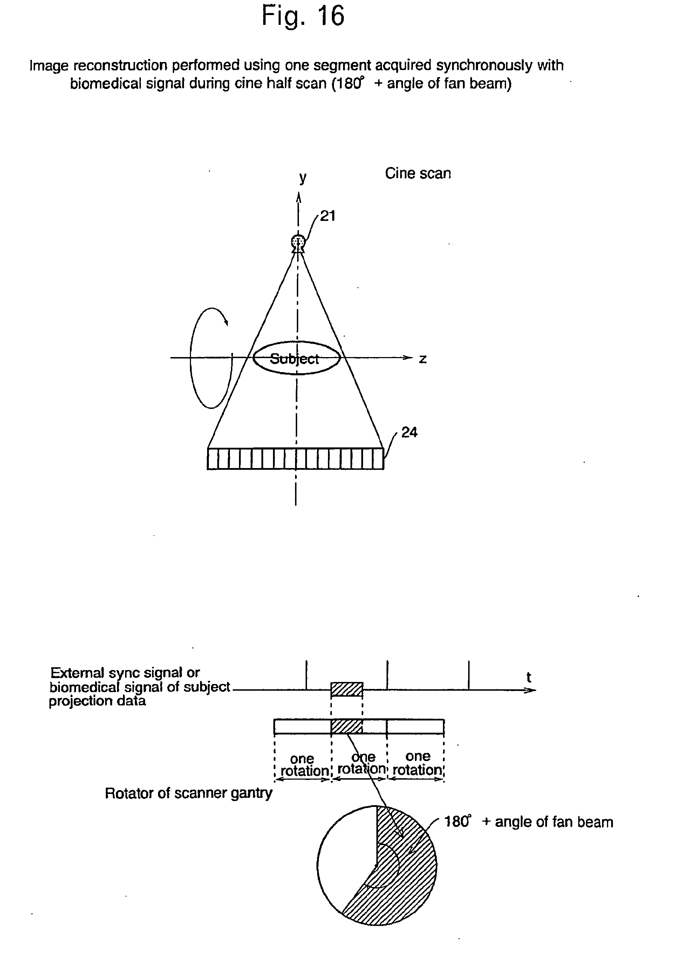

Conventionally, in X-ray CT apparatuses including a two-dimensional X-ray area detector that has a matrix structure and is represented by a multi-array X-ray detector or a flat-panel X-ray detector, a subject is helically scanned as shown in Fig. 12. One segment that is composed of projection data items acquired during a helical scan, that is synchronous with a certain phase of an external synchronizing (sync) signal or a biomedical signal of a subject, and that corresponds to projection data items acquired during a half scan is, as shown in Fig. 13, used to reconstruct an image. Otherwise, as shown in Fig. 14 and Fig. 15, a plurality of segments (composed of projection data items) corresponding to projection data items acquired during a half scan (180° + the angle of a fan beam falling on the detector) or during a 360° full scan are combined or weighted and then summated in order to reconstruct an image (refer to, for example, Japanese Unexamined Patent Publication No. 2004-275440 (P. 3 and 5, Figs. 1, 2, 8, and 9))

However, in the above case, the cycle of a biomedical signal, the rotating speed for one scan, and the moving speed at which a subject is moved in a z direction during a helical scan must be optimized. A fluctuation in the cycle of the biomedical signal leads to deterioration of image quality, and therefore poses a problem. If the moving speed at which a subject is moved in the z direction remains constant, the fluctuation in the cycle of the biomedical signal cannot be coped with, and therefore poses a problem.

In the X-ray CT apparatus including a two-dimensional X-ray area detector represented by a multi-array X-ray detector or a flat-panel X-ray detector, the two-dimensional X-ray area detector is wide in a z direction. Moreover, since the two-dimensional X-ray area detector is composed of many detector arrays, the width in the z direction of each detector array is small. This leads to a high resolution in the z direction. Consequently, an angle of X-irradiation in the z direction gets larger, and the conical angle of an X-ray cone beam gets larger. In other words, a range in the z direction which can be radiographed by scanning one position in the z direction during a conventional (axial) scan or a cine scan is getting wider. Consequently, part of a subject or organs thereof are likely to be radiographed during the conventional (axial) scan or cine scan performed at one position in the z direction without the necessity of a helical scan. Due to these backgrounds, the significance of the conventional (axial) scan or cine scan will presumably grow.

Therefore, the present invention seeks to provide an X - ray CT apparatus that, when adapted to an X-ray CT apparatus including a two-dimensional area X-ray detector which has a matrix structure and which is represented by a multi-array X-ray detector or a flat-panel X-ray detector, and used to perform a conventional (axial) scan or a cine scan, three-dimensionally displays tomographic images that enjoy improved image quality, that is, a high temporal resolution and that show states of a subject synchronized with an external sync signal or a biomedical signal, displays a three-dimensional tomographic image that enjoys a high temporal resolution and that shows states of a subject synchronized with a plurality of phases of the external sync signal or biomedical signal, or displays a four-dimensional tomographic image that enjoys a high temporal resolution and that shows states of a subject synchronized with the external sync signal or biomedical signal.

The present invention provides an X-ray CT apparatus or an X-ray CT method that, when adapted to or implemented in an X-ray CT apparatus including a two-dimensional X-ray area detector, and used to perform a conventional (axial) scan or a cine scan, three-dimensionally displays tomographic images, which enjoy a high temporal resolution, by reconstructing a three-dimensional image using a plurality of segments, which is sampled synchronously with an external signal or a biomedical signal from data of a reconstructed three-dimensional image or from projection data items detected during a plurality of scans and which corresponds to projection data items detected during a 360° full scan or during a half scan of rotating the X-ray tube by an angle of 180° plus the angle of a fan-shaped beam falling on the detector, or four-dimensionally displays a plurality of three-dimensional images by switching the images sequentially synchronously with a plurality of phases of the external signal or biomedical signal.

According to the first aspect of the present invention, there is provided an X-ray CT apparatus including: an X-ray data acquisition means for rotating an X-ray generator and a two-dimensional X-ray area detector, which has a matrix structure, is represented by a multi-array X-ray detector or a flat-panel X-ray detector, and is opposed to the X-ray generator in order to detect X-rays, about a center of rotation located between the X-ray generator and two-dimensional X-ray area detector so as to acquire X-ray projection data items of X-rays transmitted by a subject lying down between the X-ray generator and two-dimensional X-ray area detector; an image reconstruction means for reconstructing an image using the projection data items acquired by the X-ray data acquisition means; an image display means for displaying the reconstructed tomographic image; an external signal input means for receiving an external input signal; and a phase data input means for attaining synchronization with an external sync signal. Herein, the image reconstruction means samples X-ray detector data items, which are acquired synchronously with a certain phase of the external input signal, from those acquired during cine scans so as to reconstruct an image.

In the X-ray CT apparatus according to the first aspect of the present invention, a plurality of segments including projection data items detected synchronously with a certain phase of an external input sync signal is sampled from projection data items detected during a plurality of cine scans, whereby projection data items corresponding to those detected during a 360° full scan or during a half scan of rotating the X-ray tube by an angle of 180° plus an angle of a fan-shaped beam falling on the detector are sampled. A scanning/rotating speed should merely be designated to permit the sampling. The projection data items are used to reconstruct an image. This results in a tomographic image enjoying a temporal resolution equivalent to one segment.

According to the second aspect of the present invention, there is provided an X-ray CT apparatus including: an X-ray data acquisition means for rotating an X-ray generator and a two-dimensional X-ray area detector, which has a matrix structure, is represented by a multi-array X-ray detector or a flat-panel X-ray detector, and is opposed to the X-ray generator in order to detect X-rays, about a center of rotation located between the X-ray generator and two-dimensional X-ray area detector so as to acquire X-ray projection data items of X-rays transmitted by a subject lying down between the X-ray generator and two-dimensional X-ray area detector; an image reconstruction means for reconstructing an image using the X-ray projection data items acquired by the X-ray data acquisition means; an image display means for displaying the reconstructed tomographic image; a biomedical signal input means for receiving a biomedical signal; and a phase data input means for attaining synchronization with the biomedical signal. Herein, the image reconstruction means samples X-ray detector data items, which are acquired synchronously with a certain phase of a biomedical signal, from those acquired during cine scans so as to reconstruct a three-dimensional image.

In the X-ray CT apparatus according to the second aspect, a plurality of segments including projection data items detected synchronously with a certain phase of a biomedical signal is sampled from projection data items detected during a plurality of cine scans, whereby projection data items corresponding to those detected during a 360° full scan or during a half scan of rotating the X-ray tube by an angle of 180° plus an angle of a fan-shaped beam falling on the detector are sampled. A scanning/rotating speed should merely be designated to permit the sampling. The projection data items are used to reconstruct an image. This results in a tomographic image enjoying a temporal resolution equivalent to one segment. Moreover, when three-dimensional image reconstruction is adopted, a high-quality tomographic image is produced while being little affected by artifacts attributable to a portion of an X-ray beam falling outside in the z direction the X-ray detector arrays.

According to the third aspect of the present invention, there is provided an X-ray CT apparatus identical to the X-ray CT apparatus according to the first or second aspect except that the image reconstruction means reconstructs an image by sampling X-ray detector data items that are acquired synchronously with a certain phase of a biomedical signal and that correspond to those acquired during a cine scan of rotating the X-ray tube by an angle of 180° plus the angle of a fan beam falling on the detector.

In the X-ray CT apparatus according to the third aspect, a plurality of segments including projection data items detected synchronously with a certain phase of a biomedical signal is sampled from projection data items detected during a plurality of cine scans, whereby projection data items corresponding to those detected during a half scan of rotating the X-ray tube by an angle of 180° plus the angle of a fan beam falling on the detector can be sampled. A scanning/rotating speed should merely be designated to permit the sampling. The projection data items are used to reconstruct an image. This results in a tomographic image enjoying a temporal resolution equivalent to one segment.

According to the fourth aspect of the present invention, there is provided an X-ray CT apparatus identical to the X-ray CT apparatus according to any of the first to third aspects except that the image reconstruction means samples a plurality of segments of X-ray detector data items, which is acquired synchronously with a certain phase of a biomedical signal, from those acquired during cine scans. At this time, projection data items produced from the X-ray detector data items are combined or weighted and summated so that view angles at which the X-ray detector data items are acquired will come to 360° or an angle of 180° plus the angle of a fan beam falling on the detector, whereby an image is reconstructed.

In the X-ray CT apparatus according to the fourth aspect, a plurality of segments including projection data items detected synchronously with a certain phase of a biomedical signal is sampled from projection data items detected during a plurality of cine scans, whereby projection data items corresponding to those detected during a 360° full scan or during a half scan of rotating the X-ray tube by an angle of 180° plus the angle of a fan beam falling on the detector can be sampled. A scanning/rotating speed should merely be designated to permit the sampling. The projection data items are used to reconstruct an image. This results in a tomographic image enjoying a temporal resolution equivalent to one segment. Since projection data items detected during one scan are grouped into a plurality of segments, the temporal resolution of a tomographic image improves.

According to the fifth aspect of the present invention, there is provided an X-ray CT apparatus identical to the X-ray CT apparatus according to any of the first to fourth aspects except that the image reconstruction means samples X-ray detector data items, which are acquired synchronously with certain phases of a plurality of biomedical signals, from X-ray detector data items acquired during cine scans so as to reconstruct an image.

In the X-ray CT apparatus according to the fifth aspect, a plurality of segments including projection data items detected synchronously with certain phases of a plurality of sync signals is sampled from projection data items detected during a plurality of cine scans, whereby projection data items corresponding to those detected during a 360° full scan or during a half scan of rotating the X-ray tube by an angle of 180° plus the angle of a fan beam falling on the detector can be sampled. A scanning/rotating speed should merely be designated to permit the sampling. The projection data items are used to reconstruct an image. This results in a tomographic image enjoying a temporal resolution equivalent to one segment.

According to the sixth aspect of the present invention, there is provided an X-ray CT apparatus identical to the X-ray CT apparatus according to any of the first to fifth aspects except that when X-ray detector data items acquired synchronously with a certain phase of a biomedical signal are sampled from those acquired during cine scans, if a temporal resolution that is a time width during which data acquisition is performed once is restricted, the X-ray data acquisition means optimizes the rotating speed of a data acquisition apparatus so that data can be acquired with a small number of rotations of the data acquisition apparatus.

In the X-ray CT apparatus according to the sixth aspect, if a temporal resolution should be decreased because of the property of a biomedical signal, the number of segments is increased, and a plurality of segments including projection data items detected synchronously with a phase of the biomedical signal is sampled from projection data items detected during a plurality of cine scans. Thus, projection data items corresponding to those detected during a 360° full scan or during a half scan of rotating the X-ray tube by an angle of 180° plus the angle of a fan beam falling on the detector can be sampled. A scanning/rotating speed should merely be designated to permit the sampling. The projection data items are used to reconstruct an image. This results in a tomographic image enjoying a temporal resolution equivalent to one segment.

According to the seventh aspect of the present invention, there is provided an X-ray CT apparatus identical to the X-ray CT apparatus according to any of the first to sixth aspects except that the image reconstruction means applies a z-direction filter to projection data during image reconstruction so that the slice thickness of a tomographic image to be reconstructed will be an arbitrary slice thickness which is in the center of image reconstruction larger than the width in the z direction, that is, the direction of arrays of the multi-array X-ray detector.

In the X-ray CT apparatus according to the seventh aspect, a plurality of segments including projection data items detected synchronously with a certain phase of an external input sync signal or a biomedical signal is sampled from projection data items detected during a plurality of cine scans, whereby projection data items corresponding to those detected during a 360° full scan or during a half scan of rotating the X-ray tube by an angle of 180° plus the angle of a fan beam falling on the detector can be sampled. A scanning/rotating speed should merely be designated to permit the sampling. The projection data items are used to reconstruct an image. This results in a tomographic image enjoying a temporal resolution equivalent to one segment. In addition, when image quality must be improved from the viewpoint of reduction of artifacts or noises, the z-direction filter applied to projection data items can be used to control the artifacts or noises or a slice thickness.

According to the eighth aspect of the present invention, there is provided an X-ray CT apparatus identical to the X-ray CT apparatus according to any of the first to sixth aspects except that the image reconstruction means applies a z-direction filter to image data after completion of image reconstruction so that the slice thickness of a tomographic image to be reconstructed will be an arbitrary slice thickness which is in the center of image reconstruction larger than the width in the z direction, that is, the direction of detector arrays of the multi-array X-ray detector.

In the X-ray CT apparatus according to the eighth aspect, a plurality of segments including projection data items detected synchronously with a certain phase of an external input sync signal or a biomedical signal is sampled from projection data items detected during a plurality of cine scans, wherein projection data items corresponding to those detected during a 360° full scan or during a half scan of rotating the X-ray tube by an angle of 180° plus the angle of a fan beam falling on the detector. A scanning/rotating speed should merely be designated to permit the sampling. The projection data items are used to reconstruct an image. This results in a tomographic image enjoying a temporal resolution equivalent to one segment. In addition, when image quality must be improved from the viewpoint of reduction of artifacts or noises, the z-direction filter applied to image data can be used to control the artifacts or noises or a slice thickness.

According to the ninth aspect of the present invention, there is provided an X-ray CT apparatus identical to the X-ray CT apparatus according to any of the first to eighth aspects except that the image reconstruction means reconstructs an image so that the slice position of a tomographic image will be different from a position on a z axis, on which a center of rotation is present, extending from a center position of each of the detector arrays included in the multi-array X-ray detector.

In the X-ray CT apparatus according to the ninth aspect, a plurality of segments including projection data items detected synchronously with a certain phase of an external input sync signal or a biomedical signal is sampled from projection data items detected during a plurality of cine scans, whereby projection data items corresponding to those detected during a 360° full scan or during a half scan of rotating the X-ray tube by an angle of 180° plus the angle of a fan beam falling on the detector. A scanning/rotating speed should merely be designated to permit the sampling. The projection data items are used to reconstruct an image. This results in a tomographic image enjoying a temporal resolution equivalent to one segment. In particular, when three-dimensional image reconstruction is adopted as an image reconstruction technique to be used in combination with a cine scanning method, a tomographic image can be reconstructed relative to any position in the z direction irrespective of the position of a detector array in the z direction. In other words, a slice position in the z direction can be arbitrarily controlled as it is when a conventional helical scanning method is adopted. Tomographic images expressing slices that overlap in the z direction can be reconstructed. Consequently, image quality ensured for three-dimensional display, MPR display, or MIP display can be improved.

According to the tenth aspect of the present invention, there is provided an X-ray CT apparatus identical to the X-ray CT apparatus according to any of the first to ninth aspects except that the image reconstruction means reconstructs an image so that the inter-slice spacing of a tomographic image will be different from the spacing between adjoining ones of the detector arrays, which are included in the multi-array X-ray detector, on the z axis on which a center of rotation is present.

In the X-ray CT apparatus according to the tenth aspect, a plurality of segments including projection data items detected synchronously with a certain phase of an external input sync signal or a biomedical signal is sampled from projection data items detected during a plurality of cine scans, whereby projection data items corresponding to those detected during a 360° full scan or during a half scan of rotating the X-ray tube by an angle of 180° plus the angle of a fan beam falling on the detector are sampled. A scanning/rotating speed should merely be designated to permit the sampling. The projection data items are used to reconstruct an image. This results in a tomographic image enjoying a temporal resolution equivalent to one segment. In particular, when three-dimensional image reconstruction is adopted as an image reconstruction technique to be used in combination with a cine scanning method, a tomographic image can be reconstructed relative to any position in a z direction irrespective of the position of a detector array in the z direction. In other words, a slice position in the z direction can be arbitrarily controlled as it is when a conventional helical scanning method is adopted. Tomographic images representing slices that overlap in the z direction can be reconstructed, and image quality ensured for three-dimensional display, MPR display, or MIP display can be improved. Tomographic images of slices having any spacing in the z direction between adjoining ones can be reconstructed, and image quality ensured for three-dimensional display, MPR display, or MIP display can be improved.

According to the eleventh aspect of the present invention, there is provided an X-ray CT apparatus identical to the X-ray CT apparatus according to any of the first to tenth aspects except that the image reconstruction means reconstructs an image with the slice thickness, slice position, and inter-slice spacing of a tomographic image set to arbitrary values.

In the X-ray CT apparatus according to the eleventh aspect, a plurality of segments including projection data items detected synchronously with a certain phase of an external input sync signal or a biomedical signal is sampled from projection data items detected during a plurality of cine scans, whereby projection data items corresponding to those detected during a 360° full scan or a half scan of rotating the X-ray tube by an angle of 180° plus the angle of a fan beam falling on the detector can be sampled. A scanning/rotating speed should merely be designated to permit the sampling. The projection data items are used to reconstruct an image. This results in a tomographic image enjoying a temporal resolution equivalent to one segment. In particular, when three-dimensional image reconstruction is adopted as an image reconstruction technique to be used in combination with a cine scanning method, a tomographic image can be reconstructed relative to any position in a z direction irrespective of the position of a detector array in the z direction. In other words, a slice position in the z direction can be arbitrarily controlled as it is when a conventional helical scanning method is adopted. Tomographic images representing slices that overlap in the z direction can be reconstructed, and image quality ensured for three-dimensional display, MPR display, or MIP display can be improved. Moreover, a slice thickness can be arbitrarily controlled. Consequently, freedom in reconstructing an image is intensified.

According to the twelfth aspect of the present invention, there is provided an X-ray CT apparatus identical to the X-ray CT apparatus according to any of the second to eleventh aspects except that the data acquisition means performs a cine scan at a plurality of different positions in the z direction that is a direction in which a cradle included in a table is advanced, and that the image reconstruction means reconstructs an image by sampling X-ray beam data items of X-rays, which are irradiated in the same direction and pass through pixels on a field of view, and X-ray beam data items of opposed X-rays from X-ray beam data items contained in X-ray detector data items acquired during a plurality of cine scans performed at the respective positions in the z direction.

In the X-ray CT apparatus according to the twelfth aspect, when a cine scan is performed at a plurality of positions in the z direction, if ranges to be radiographed during the cine scans adjoin in the z direction or overlap, X-ray beam data items of X-rays irradiated in the same direction and X-ray beam data items of opposed X-rays are sampled from projection data items detected during the cine scans performed at the respective positions in the z direction, and used to reconstruct a three-dimensional image. Consequently, noises or artifacts in a tomographic image can be reduced.

According to the thirteenth aspect of the present invention, there is provided an X-ray CT apparatus identical to the X-ray CT apparatus according to any of the first to twelfth aspects except that the X-ray data acquisition means acquires X-ray detector data items during a cine scan during which X-rays are irradiated only at the timing synchronous with a certain phase of a biomedical signal, and the image reconstruction means samples X-ray detector data items, which represent a region to which the X-rays are irradiated, so as to reconstruct an image.

In the X-ray CT apparatus according to the thirteenth aspect, initiation of X-irradiation (ON) or termination thereof (OFF) is synchronized with an external input signal or a biomedical signal. Consequently, X-irradiation is minimized or reduced. This leads to a decrease in a patient dose.

According to the fourteenth aspect of the present invention, there is provided an X-ray CT apparatus identical to the X-ray CT apparatus according to any of the first to twelfth aspects except that the X-ray data acquisition means acquires X-ray detector data items during a cine scan during which a larger number of X-rays is irradiated at the timing synchronous with a certain phase of a biomedical signal and a smaller number of X-rays is irradiated at the other timings, and that the image reconstruction means samples X-ray detector data items, which represents a region to which the larger number of X-rays is irradiated, so as to reconstruct an image.

In the X-ray CT apparatus according to the fourteenth aspect, increase or decrease of an exposure is synchronized with an external input signal or a biomedical signal. Consequently, X-irradiation is minimized or reduced. This leads to a decrease in a patient dose.

According to the fifteenth aspect of the present invention, there is provided an X-ray CT apparatus identical to the X-ray CT apparatus according to any of the first to fourteenth aspects except that the image reconstruction means reconstructs a tomographic image that shows states of a subject synchronized with a plurality of phases of a biomedical signal.

In the X-ray CT apparatus according to the fifteenth aspect, synchronous radiography is achieved appropriately by attaining synchronization with a plurality of phases of a biomedical signal.

According to the sixteenth aspect of the present invention, there is provided an X-ray CT apparatus identical to the X-ray CT apparatus according to the fifteenth aspect except that the image display means achieves dynamic display by time-sequentially displaying a tomographic image that shows states of a subject synchronized with a plurality of phases of a biomedical signal.

In the X-ray CT apparatus according to the sixteenth aspect, a plurality of tomographic images showing states of a subject synchronized with respective phases is reconstructed so that a phase change can be dynamically displayed.

According to the seventeenth aspect of the present invention, there is provided an X-ray CT apparatus identical to the X-ray CT apparatus according to any of the first to fourteenth aspects except that the image reconstruction means reconstructs a plurality of tomographic images expressing sections juxtaposed in the z direction and showing states of a subject synchronized with a plurality of phases of a biomedical signal.

In the X-ray CT apparatus according to the seventeenth aspect, successive tomographic images expressing sections juxtaposed in the z direction and showing states of a subject synchronized with a plurality of biomedical signals, that is, a three-dimensional image is reconstructed. Synchronous three-dimensional image reconstruction can be achieved more accurately.

According to the eighteenth aspect of the present invention, there is provided an X-ray CT apparatus identical to the X-ray CT apparatus according to the seventeenth aspect except that the image display means achieves dynamic three-dimensional display by time-sequentially displaying a tomographic image that shows states of a subject synchronized with a plurality of phases of a biomedical signal.

In the X-ray CT apparatus according to the eighteenth aspect, successive tomographic images expressing sections juxtaposed in the z direction and showing states of a subject synchronized with a plurality of biomedical signals, that is, a three-dimensional image is reconstructed. Consequently, synchronous three-dimensional image reconstruction can be achieved more accurately. Moreover, when a plurality of three-dimensional images showing states of a subject synchronized with a plurality of phases is reconstructed, a phase change can be dynamically displayed.

According to the nineteenth aspect of the present invention, there is provided an X-ray CT apparatus identical to the X-ray CT apparatus according to any of the second to eighteenth aspects except that the biomedical signal input means receives a heartbeat signal as a first biomedical signal.

In the X-ray CT apparatus according to the nineteenth aspect, the heartbeat signal is received as the first biomedical signal. Consequently, heartbeat-synchronous tomography or heartbeat-synchronous three-dimensional radiography can be achieved.

According to the twentieth aspect of the present invention, there is provided an X-ray CT apparatus identical to the X-ray CT apparatus according to any of the second to nineteenth aspects except that the biomedical signal input means receives a respiratory signal as a second biomedical signal.

In the X-ray CT apparatus according to the twentieth aspect, the respiratory signal is received as the second biomedical signal. Consequently, respiration-synchronous tomography or respiration-synchronous three-dimensional radiography can be achieved.

According to an X-ray CT apparatus or an X-ray CT image reconstruction method in which the present invention is implemented, when an X-ray CT apparatus including a two-dimensional area X-ray detector that has a matrix structure and is represented by a multi-array X-ray detector or a flat-panel X-ray detector performs a conventional (axial) scan or a cine scan, tomographic images that enjoy improved image quality, that is, a high temporal resolution, and that show states of a subject synchronized with an external sync signal or a biomedical signal are three-dimensionally displayed. Otherwise, a three-dimensional tomographic image that enjoys a high temporal resolution and shows states of a subject synchronized with a plurality of phases of the external sync signal or biomedical signal is displayed. Otherwise, a four-dimensional tomographic image that enjoys a high temporal resolution and shows states of a subject synchronized with the external sync signal or biomedical signal is displayed.

The invention will now be described in greater detail, by way of example, with reference to the drawings, in which:-

Fig. 1 is a block diagram showing an X-ray CT apparatus in accordance with an embodiment of the present invention.

Fig. 2 is an explanatory diagram showing the rotation of an X-ray generator (X-ray tube) and a multi-array X-ray detector.

Fig. 3 is a flowchart outlining actions to be performed in the X-ray CT apparatus in accordance with the embodiment of the present invention.

Fig. 4 is a flowchart describing preprocessing.

Fig. 5 is a flowchart describing three-dimensional image reconstruction.

Fig. 6 includes conceptual diagrams showing projection of lines on a field of view in a direction of X-ray transmission.

Fig. 7 is a conceptual diagram showing lines projected on a detector surface.

Fig. 8 is a conceptual diagram showing projection data items Dr(view,x,y) projected on the field of view.

Fig. 9 is a conceptual diagram showing back projection pixel data items D2 associated with pixels constituting the field of view.

Fig. 10 is an explanatory diagram showing production of back projection data items D3 having back projection pixel data items D2 added thereto pixel by pixel.

Fig. 11 includes conceptual diagrams showing projection of lines on a circular field of view in a direction of X-ray transmission.

Fig. 12 is a conceptual diagram showing a helical scan.

Fig. 13 is an explanatory diagram showing image reconstruction performed using one segment of data items acquired synchronously with a biomedical signal during a helical half scan (180°+fan-beam angle).

Fig. 14 is an explanatory diagram showing image reconstruction performed using three segments corresponding to data items acquired during the helical half scan (180°+fan-beam angle).

Fig. 15 is an explanatory diagram showing image reconstruction performed using four segments corresponding to data items acquired during a helical full scan (360°).

Fig. 16 shows one segment acquired synchronously with a biomedical signal during a cine half scan (180°+fan-beam angle).

Fig. 17 shows three segments corresponding to data items acquired during a cine half scan (180°+fan-beam angle).

Fig. 18 shows four segments corresponding to data items acquired during a cine full scan (360°).

Fig. 19 shows a method of receiving the synchronizing phase of an external sync signal or a biomedical signal.

Fig. 20 shows the synchronizing phase of an external sync signal or a biomedical signal and switching of initiation (ON) and termination (OFF) of X-irradiation or increase and decrease of an X-ray dose.

Fig. 21 shows a control sequence of controlling a cine scan synchronously with an external sync signal or a biomedical signal.

Fig. 22 shows reconstruction of a plurality of tomographic images using data items detected by a multi-array X-ray detector 24 during a cine scan.

Fig. 23 shows reconstruction of successive tomographic images as a time-sequential three-dimensional tomographic image using three segments corresponding to data items acquired synchronously with a signal during a cine half scan.

Fig. 24 shows reconstruction of successive time-sequential three-dimensional tomographic images using three segments corresponding to data items acquired synchronously with a plurality of phases during a cine half scan.

Fig. 25 shows weighting of projection data items contained in respective segments.

The present invention will be described below by taking an illustrated embodiment for instance. Noted is that the present invention will not be limited to the embodiment.

Fig. 1 is a block diagram showing the configuration of an X-ray CT apparatus in accordance with an embodiment of the present invention. The X-ray CT apparatus 100 includes an operator console 1, a radiographic table 10, and a scanner gantry 20.

The operator console 1 includes an input device 2 that receives an operator's input, a central processing unit 3 that executes preprocessing, image reconstruction, and post-processing, a data collection buffer 5 that collects X-ray detection data items acquired by the scanner gantry 20, a monitor 6 on which a tomographic image reconstructed based on projection data items produced by preprocessing the X-ray detector data items is displayed, and a storage device 7 in which programs, X-ray detector data items, projection data items, and X-ray tomographic images are stored.

The radiographic table 10 includes a cradle 12 that carries a subject, who lies down on the cradle 12, into or out of the bore of the scanner gantry 20. The cradle 12 is raised, lowered, or rectilinearly moved by a motor incorporated in the radiographic table 10.

The scanner gantry 20 includes an X-ray tube 21, an X-ray controller 22, a collimator 23, a multi-array X-ray detector 24, a data acquisition apparatus (DAS) 25, a rotator controller 26 that controls the X-ray tube 21 rotating about the body axis of a subject, and a control unit 29 that transfers control signals to or from the operator console 1 and radiographic table 10. Moreover, a scanner gantry tilt controller 27 allows the scanner gantry 20 to tilt forward or backward at approximately $\pm$ 30° with respect to a z direction.

n external sync signal or a plurality of biomedical signals are transferred to the control unit 29 via an input interface 31 that receives the external sync signal or one or a plurality of biomedical signals.

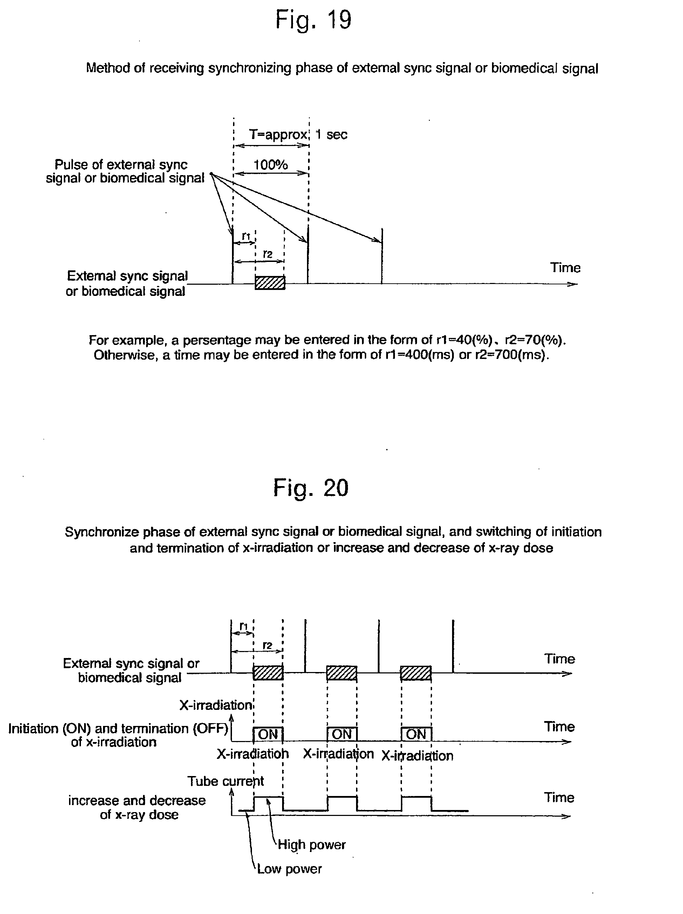

Operator's keystrokes may be adopted as a means for entering a synchronizing (sync) timing that is a phase synchronous with an external sync signal or a biomedical signal. As shown in Fig. 19, a percentage may be entered in the form of, for example, r1=40 (%) or r2=70 (%). Otherwise, a time may be entered in the form of, for example, r1=400 (ms) or r2=700 (ms).

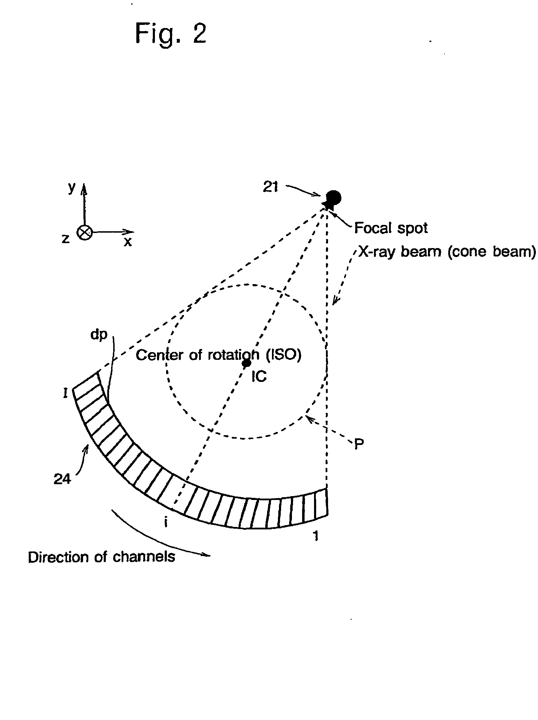

Fig. 2 is an explanatory diagram sowing the geometric disposition of the X-ray tube 21 and multi-array X-ray detector 24.

The X-ray tube 21 and multi-array detector 24 rotate about a center of rotation IC. Assuming that a vertical direction is a y direction, a horizontal direction is an x direction, and a direction of table advancement perpendicular to the x and y directions is a z direction, a rotation plane on which the X-ray tube 21 and multi-array X-ray detector 24 are rotated is an xy plane. Moreover, a direction of movement in which the cradle 12 moves is the z direction.

The X-ray tube 21 generates an X-ray beam that is called a cone beam CB. When the direction of the center axis of the cone beam CB is parallel to the y direction, it is said that the X-ray tube 21 is set to a view angle 0°.

The multi-array X-ray detector 24 includes, for example, 256 X-ray detector arrays. Moreover, each of the X-ray detector arrays includes, for example, 1024 X-ray detector channels.

After X-rays are irradiated, projection data items are produced by the multi-array X-ray detector 24, and then analog-to-digital converted by the DAS 25. The resultant data items are transferred to the data collection buffer 5 via a slip ring 30. The data items transferred to the data collection buffer 5 are treated by the central processing unit 3 according to a program read from the storage device 7. Consequently, a tomographic image is reconstructed and displayed on the monitor 6. According to the present invention, a cine scan is performed. During the cine scan, the X-ray tube 21 and multi-array X-ray detector 24 are rotated about a subject, and data acquisition is performed with the cradle 12 of the radiographic table 10 fixed at a certain position in the z direction. X-ray detector data items D0(view,j,i) each identified with a view angle view, a detector array number j, and a channel number i and appended with data of a position in the z direction of rectilinear table movement, Ztable(view), are acquired.

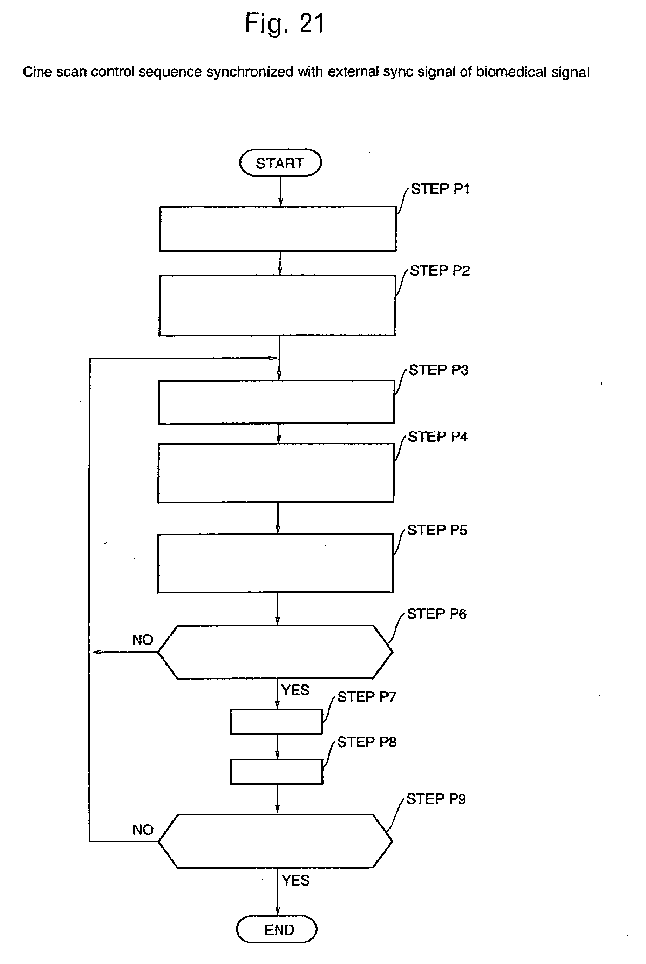

Fig. 21 is a flowchart describing a control sequence of controlling a cine scan synchronously with an external sync signal or a biomedical signal.

At step P1, the cycle T of an external sync signal or a biomedical signal shown in Fig. 19 is measured. In particular, when the biomedical signal is employed, the cycle T varies. A mean of several recent cycles is adopted.

At step P2, if a percentage is entered as each of synchronous phase timings r1 and r2 (see Fig. 19), the percentages are transformed into absolute values (ms) relevant to the cycle T according to the expressions R1=r1·T and R2=r2·T.

At step P3, after preparations have been made for data acquisition to be performed during cine scans, input of an external sync signal or a biomedical signal is awaited, and the X-ray tube 21 and multi-array X-ray detector 24 are rotated about a subject. The cradle 12 of the radiographic table 10 is fixed at a certain position in the z direction. Data of the position in the z direction of rectilinear table movement, Ztable(view), is appended to X-ray detector data items D0(view,j,i) each identified with a view angle view, a detector array number j, and a channel number i. Thus, X-ray detector data items are acquired.

At step P4, the elapse of a time r1 (ms) or R1 (ms) after input of an external sync signal or a biomedical signal is awaited, and X-irradiation is initiated or set to a high power mode. When X-irradiation is initiated or set to the high power mode, a flag indicating that X-irradiation is initiated is set in each X-ray detector data.

At step P5, the elapse of a time r2 (ms) or R2 (ms) after input of the external sync signal or biomedical signal is awaited, and X-irradiation is terminated or set to a low power mode. When X-irradiation is terminated or set to the low power mode, the flag indicating that X-irradiation is initiated is reset in each X-ray detector data.

At step P6, a determination is made of whether data items belonging to a required number of segments have been acquired. If not, control is passed to step P3, the next input of a signal is awaited, and data acquisition is performed. If so, control is passed to step P7.

At step P7, projection data items produced from the acquired segment or segments are used to reconstruct an image.

At step P8, the image is displayed. At this time, a tomographic image may be two-dimensionally displayed, or tomographic images expressing successive sections juxtaposed in the z direction may be three-dimensionally displayed. Otherwise, successive three-dimensional images may be displayed by time-sequentially switching phases, that is, a four-dimensional image may be displayed (four-dimensional image display).

At step P9, a determination is made of whether data items have been acquired by performing a required number of cine scans. If not, control is passed to step P3, the next input of a signal is awaited, and data acquisition is performed. If so, the sequence is terminated.

Fig. 20 shows the temporal relationship between switching of initiation and termination of X-irradiation or switching of the high power mode and low power mode of X-irradiation, which is performed at step P4 or P5, and an external sync signal or a biomedical signal.

Fig. 16 shows the timing of data acquisition to be attained in a case where an image is reconstructed using data items detected during a half scan, or specifically, projection data items detected synchronously with the external sync signal or biomedical signal during a half scan of rotating the X-ray tube by an angle of 180° plus the angle of a fan beam are sampled from projection data items detected during one scan in order to reconstruct an image.

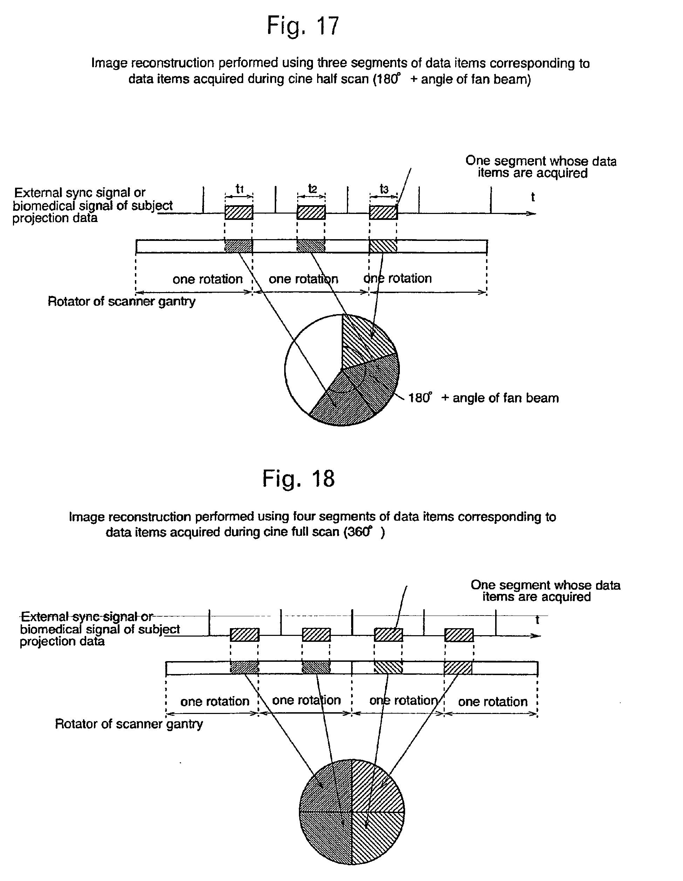

In consideration of view angles at which respective projection data items are detected, projection data items belonging to one of segments into which projection data items detected during one of a plurality of scans are grouped are sampled. Projection data items belonging to a plurality of segments are combined to produce projection data items corresponding to those detected during a half scan of rotating the X-ray tube by an angle of 180° plus the angle of a fan beam or during a 360° full scan. A tomographic image reconstructed using the projection data items enjoys a high temporal resolution that corresponds to the longest time among times t1, t2, and t3 shown in Fig. 17. Namely, when projection data items acquired during one scan are grouped into segments, the temporal resolution is improved.

As shown in Fig. 18, even when a plurality of segments is collected from projection data items detected during a plurality of scans in order to collect projection data items corresponding to those detected during a 360° full scan, a temporal resolution is improved.

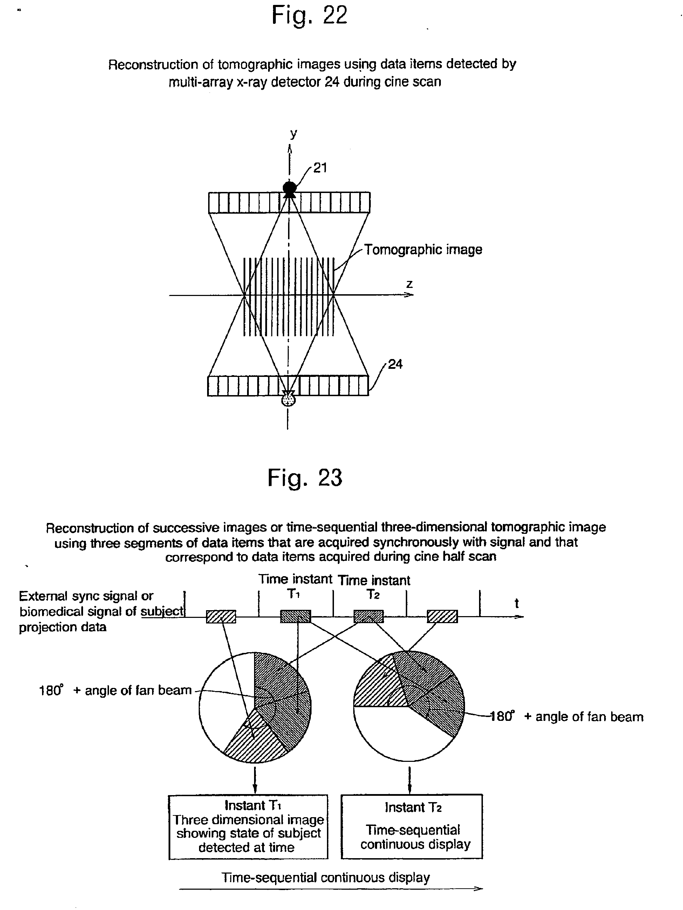

When an image is reconstructed using data items detected by the multi-array X-ray detector during a cine scan at step P8, a plurality of tomographic images is, as shown in Fig. 22, reconstructed by performing one cine scan. When three-dimensional image reconstruction is adopted, artifacts in a tomographic image are reduced. Moreover, an arbitrary number of tomographic images expressing sections located at arbitrary positions on the z axis with an arbitrary spacing between adjoining ones can be reconstructed. Furthermore, when the three-dimensional image reconstruction is adopted, if a z-direction filter is used, a tomographic image having an arbitrary slice thickness can be reconstructed.

Consequently, even when projection data items that belong to a segment and are detected synchronously with an external sync signal or a biomedical signal are sampled, or projection data items belonging to a plurality of segments are sampled from projection data items detected during a plurality of scans, a temporal resolution can be improved. Consequently, a plurality of tomographic images enjoying a high temporal resolution and showing states of a subject synchronized with the external sync signal or biomedical signal, that is, a three-dimensional tomographic image can be reconstructed.

As shown in Fig. 23, projection data items may be time-sequentially detected, and successive tomographic images may be reconstructed or displayed as a time-sequential three-dimensional tomographic image. In particular, when an image is reconstructed using a plurality of segments (three segments), every time the oldest segment data is discarded and one new segment data is adopted, a three-dimensional tomographic image is reconstructed. Thus, successive three-dimensional tomographic images are reconstructed as the time-sequential three-dimensional tomographic image. Consequently, successive three-dimensional tomographic images showing a temporal change in a certain state or a phase of a subject can be displayed as the time-sequential three-dimensional tomographic image.

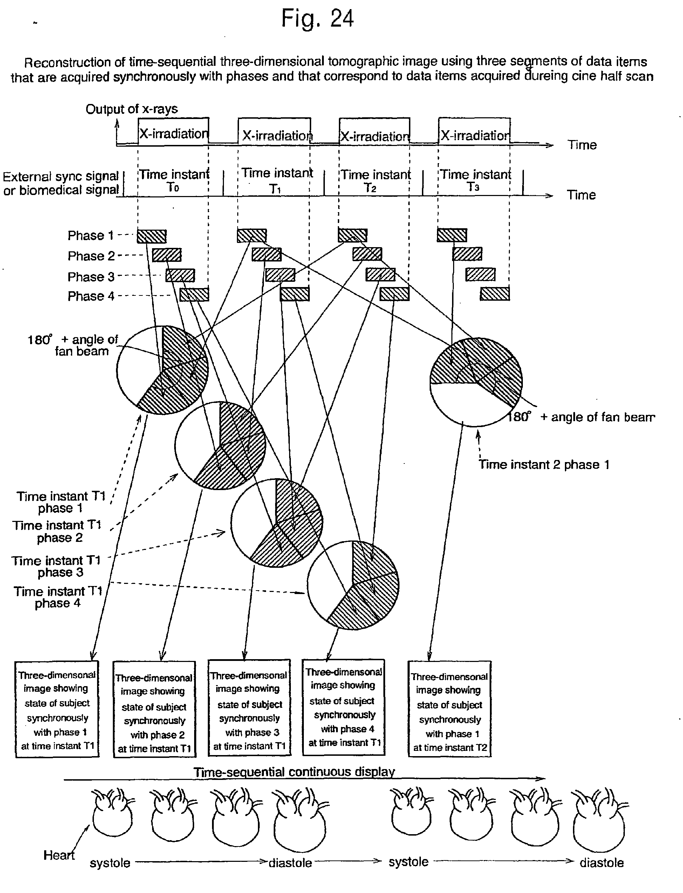

As shown in Fig. 24, three-dimensional tomographic images showing states of a subject synchronized with a plurality of phases of an external sync signal or a biomedical signal, that is, phases 1 to 4 in Fig. 24 may be reconstructed. Moreover, when successive three-dimensional tomographic images showing the states of a subject synchronized with the respective phases are time-sequentially reconstructed and displayed as successive time-sequential three-dimensional image, successive three-dimensional images showing a temporal change of the states or phases of a subject can be displayed, that is, a four-dimensional image can be displayed (four-dimensional image display).

For example, when successive three-dimensional tomographic images showing the diastole and systole of the heart synchronized with heartbeats are time-sequentially reconstructed and displayed, the diastolic and systolic phases of the heart can be observed as a time-sequential change of phases. When the time-sequential reconstruction and display of successive three-dimensional images are repeated, the diastole and systole can be repeatedly visualized.

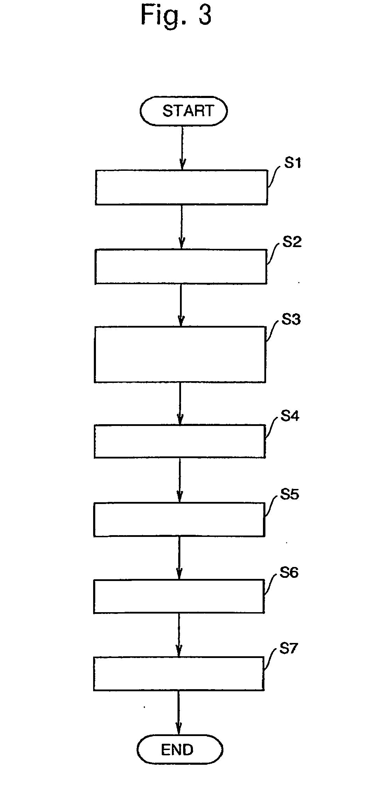

Fig. 3 is a flowchart outlining actions to be performed in order to reconstruct an image in the X-ray CT apparatus 100 in accordance with the present invention.

At step S1, first, the X-ray tube 21 and multi-array X-ray detector 24 are rotated about a subject. A cine scan is performed with the cradle 12 of the radiographic table 10 fixed at a certain position in the z direction. Data of the table position in the z direction, Ztable(view), is appended to X-ray detector data items D0(view,j,i) each identified with a view angle view, a detector array number j, and a channel number i. Thus, X-ray detector data items are acquired.

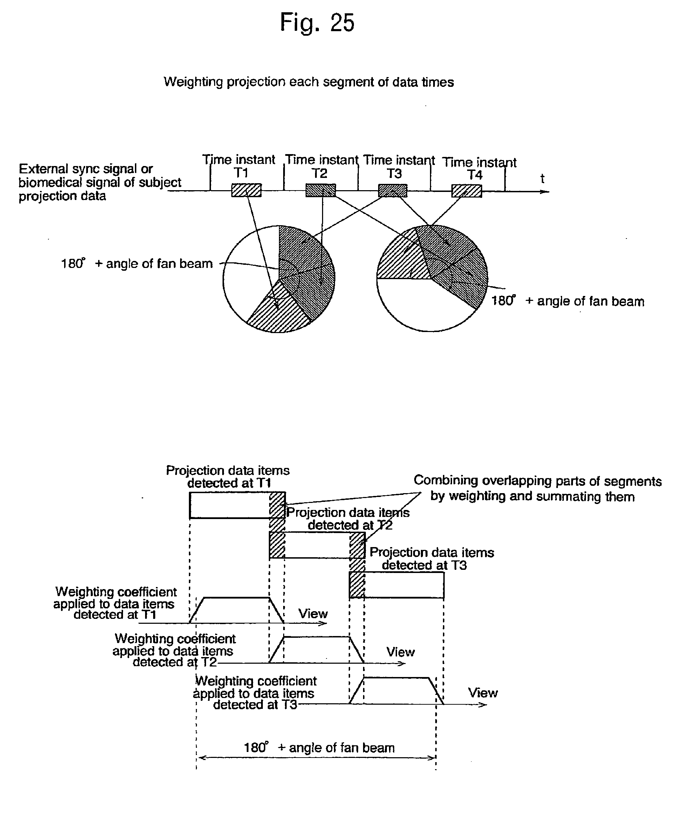

In particular, when projection data items belonging to a plurality of segments are combined or weighted and summated, the projection data items detected at respective timings are sampled so that parts of adjoining segments will be the same projection data contained in two adjoining views, that is, will overlap in a direction of time. As shown in Fig. 25, overlapping parts of segments are multiplied by a linear weighting coefficient, and the resultant projection data items are summated. Consequently, the segments of projection data items are smoothly joined, and artifacts occurring in an image portion expressed by the joint data are limited.

Moreover, if a region of a subject that should be imaged cannot be covered by scanning the subject with the cradle set at one position in the z direction, a cine scan is performed synchronously with an external sync signal or a biomedical signal at a plurality of positions in the z direction. Thus, a radiographic range in the z direction may be extended. At this time, artifacts may be intensified during scanning performed at both ends of the radiographic range in the z direction within one cine scan. Among X-ray beam data items contained in X-ray detector data items acquired by performing a cine scan at a plurality of positions in the z direction, X-ray beam data items of X-rays that are irradiated in the same direction and that pass through pixels constituting a field of view, and X-ray beam data items of opposed X-rays are sampled in order to reconstruct a three-dimensional image. In this case, an image having artifacts and noises minimized can be reconstructed.

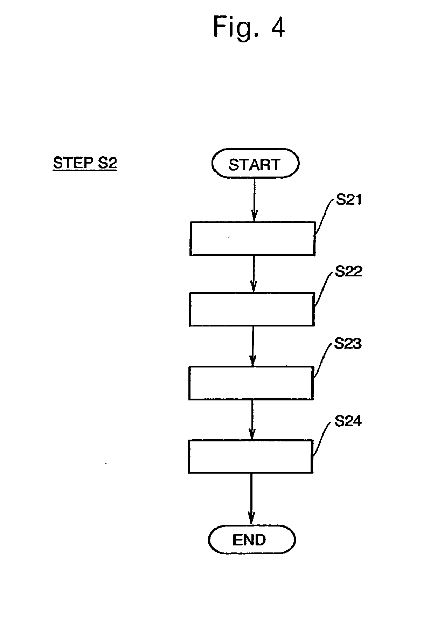

At step S2, the X-ray detector data items D0(view,j,i) are preprocessed and thus converted into projection data items. The preprocessing includes, as described in Fig. 4, offset nulling of step S21, logarithmic conversion of step S22, X-ray dose correction of step S23, and sensitivity correction of step S24.

At step S3, beam hardening correction is performed on the preprocessed projection data items D1(view,ji,i). During the beam hardening correction S3, assuming that the projection data items having undergone the sensitivity correction S24 included in the preprocessing S2 is D1(view,j,i) and data items having undergone the beam hardening correction S3 is D11(view,j,i), the beam hardening correction S3 is expressed with, for example, a polynomial as follows:

[Formula 1]

$$D11(view,j,i) =$$

$$D1(view,j,i)\cdot(B_0(j,i)+(B_1(j,i)\cdot D_1(view,j,i)+B_2(j,i)\cdot D1(view,j,i)^2)$$

At this time, the beam hardening correction can be performed on data detected by each detector array j independently of data detected by any other detector array. Consequently, if a tube voltage of a data acquisition apparatus that is one of radiographic conditions is differentiated, a difference of the X-ray energy characteristic of each detector array can be compensated.

At step S4, z filter convolution is performed in order to apply a z-direction (direction-of-arrays) filter to the projection data items D11(view,j,i) having undergone the beam hardening correction.

At step S4, a filter whose width in the direction of arrays corresponds to five arrays and which is expressed below is applied to projection data items of multi-array X-ray detector data items D11(ch,row) (where ch ranges from 1 to CH and row denotes 1 to ROW) that have been acquired at each view angle with each tube voltage and have undergone preprocessing and beam hardening correction.

$$(w_1(ch),\ w_2(ch),\ w_3(ch),\ w_4(ch),\ w_5(ch))$$

[Formula 2]

where $\displaystyle\sum_{k=1}^{5}(w_k(ch)=1$ shall be established.

Corrected detector data items D12(ch,row) are expressed as follows:

[Formula 3]

$$D12(ch,j)\sum_{k=1}^{5}(D11(ch,i-k-3)\cdot W_k(ch))$$

Incidentally, a maximum channel number shall be CH and a maximum array number shall be ROW.

[Formula 4]

$$D11(ch,-1) = D11(ch,0) = D11(ch,1)$$

$$D11(ch,ROW) = D11(ch,ROW+1) = D11(ch,ROW+2)$$

If direction-of-arrays filtering coefficients are varied depending on a channel number, a slice thickness can be controlled based on a distance from the center of image reconstruction. In general, the slice thickness of a tomographic image reconstructed with data items detected on an edge of the detector is larger than that of a tomographic image reconstructed with data items detected in the center of reconstruction thereof. Therefore, the direction-of-arrays filtering coefficients are varied depending on whether data items are detected in the center of the detector or the edge thereof. The values of the direction-of-array filtering coefficients to be applied to data items detected on channels located near the center of the detector are determined to have a large variance, and the values thereof to be applied to data items detected by channels located on the edge of the detector are determined to have a small variance. In this case, the slice thickness is nearly the same irrespective of whether a tomographic image is reconstructed using data items detected on the edge of the detector or in the center thereof.

As mentioned above, if the direction-of-arrays filtering coefficients are controlled depending on whether they are applied to data items detected on the channels located in the center of the multi-array X-ray detector 24 or data items detected on the channels located on the edge thereof, the slice thickness can be controlled depending on whether a tomographic image is reconstructed using data items detected in the center of the detector or on the edge thereof. When the slice thickness is set to a bit large value using the direction-of-arrays filter, both artifacts and noises can be reduced greatly. Consequently, the degree of reducing artifacts or noises can be controlled. In other words, the image quality of a reconstructed three-dimensional tomographic image, that is, image quality attained on the xy plane can be controlled. According to another embodiment, the direction-of-arrays (z-direction) filtering coefficients are determined to realize a de-convolution filter. In this case, a tomographic image of a small slice thickness can be constructed.

At step S5, reconstruction function convolution is performed. Specifically, data items are Fourier-transformed, applied a reconstruction function, and then inverse-Fourier-transformed. Assuming that data items having undergone the z-filter convolution are D12, data items having undergone the reconstruction function convolution are D13, and the reconstruction function to be applied is Kemel(j), the reconstruction function convolution S5 is expressed as follows:

[Formula 5]

$$D13(view,j,i) = D12(view,j,i) * Kernel(j)$$

Namely, the reconstruction function Kernel(j) can be convoluted to projection data produced by each detector array j independently of projection data produced by any other detector array. Consequently, a difference of the noise or resolution characteristic of the projection data produced by each detector array can be compensated.

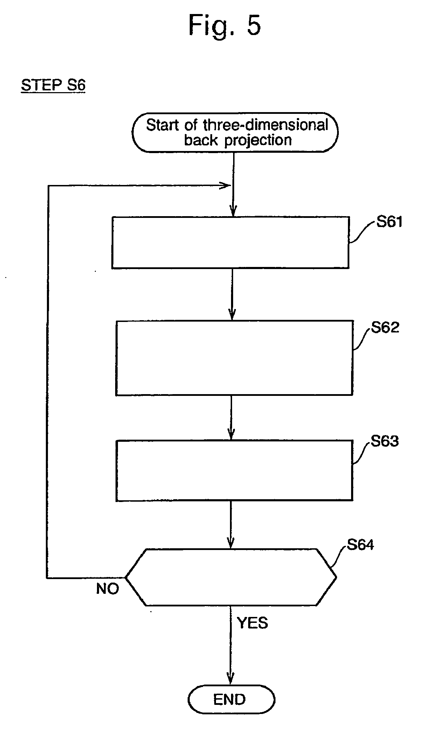

At step S6, three-dimensional back projection is performed on projection data items D13(view,j,i) having undergone reconstruction function convolution in order to produce back projection data items D3(x,y). Since the present invention adopts the cine scanning method, a three-dimensional image is reconstructed relative to a plane perpendicular to the z axis, that is, the xy plane. Hereinafter, a field of view P shall be parallel to the xy plane. The three-dimensional back projection will be described later with reference to Fig. 5.

At step S7, post-processing including image filter convolution and CT number transform is performed on the back projection data items D3(x,y,z) in order to produce tomographic image data items D31 (x,y).

Assuming that tomographic image data items having undergone the three-dimensional back projection is D31 (x,y,z), data items having undergone the image filter convolution is D32(x,y,z), and an image filter is Filter(z), the image filter convolution included in the post-processing is expressed as follows:

[Formula 6]

$$D32(x,y,z) = D32(x,y,z) * Filter(z)$$

Since the image filer can be convoluted to projection data produced by each detector array j independently of projection data produced by any other detector array, a difference of the noise or resolution characteristic of the data produced by each detector array can be compensated.

A tomographic image is then displayed on the monitor 6 according to the resultant tomographic image data items.

Fig. 5 is a flowchart describing three-dimensional back projection (step S6 in Fig. 4).

According to the present invention, a three-dimensional image is reconstructed relative to a plane perpendicular to the z axis, that is, the xy plane. A field of view P shall be parallel to the xy plane.

At step S61, one of all views needed to reconstruct a tomographic image (that is, views detected by rotating the X-ray tube 360° or an angle of 180° plus the angle of a fan beam) is selected, and projection data items Dr associated with pixels constituting the field of view P are sampled.

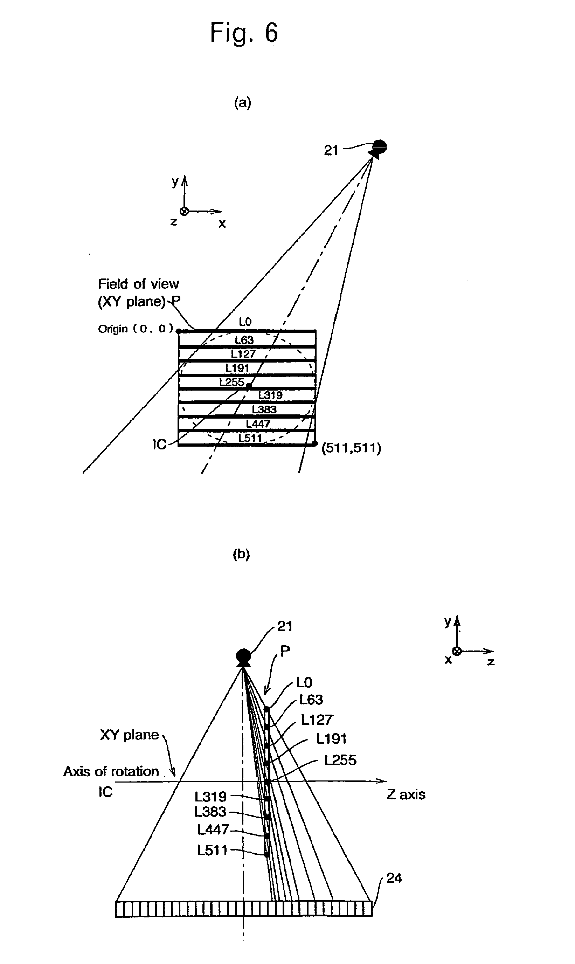

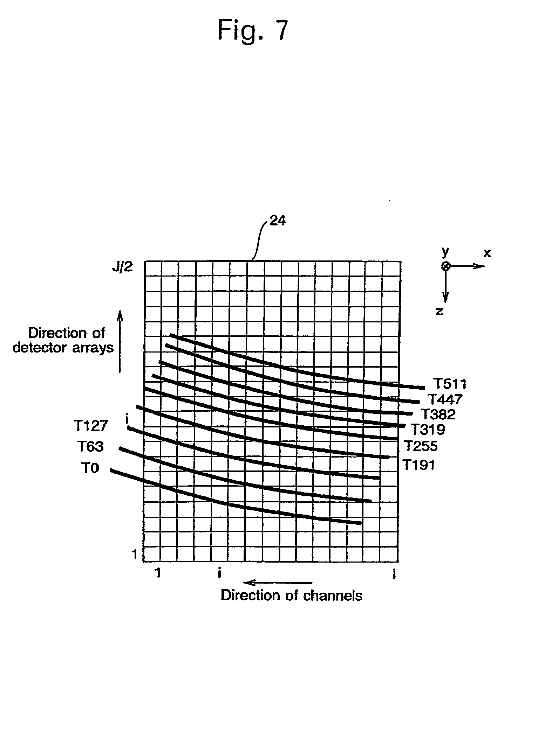

As shown in Fig. 6a and Fig. 6b, assume that the field of view P is a square field having 512 pixels lined in rows and columns and being parallel to the xy plane, and that a line of pixels L0 is parallel to the x axis and extended from a point y=0, a line of pixels L63 is extended from a point y=63, a line of pixels L127 is extended from a point y=127, a line of pixels L191 is extended from a point y=191, a line of pixels L255 is extended from a point y=255, a line of pixels L319 is extended from a point y=319, a line of pixels L383 is extended from a point y=383, a line of pixels L447 is extended from a point y=447, and a line of pixels L511 is extended from a point y=511. When the lines of pixels L0 to L511 are projected to the surface of the multi-array X-ray detector 24 in a direction of X-ray transmission, lines T0 to T511 are formed as shown in Fig. 7. Projection data items forming the lines T0 to T511 are sampled as projection data items Dr(view,x,y) representing the lines of pixels L0 to L511. Herein, x and y denote x- and y-coordinates representing each pixel in a tomographic image.

The direction of X-ray transmission is determined with the geometric positions of the focal spot in the X-ray tube 21, each pixel, and the multi-array X-ray detector 24. Since a z-coordinate z(view) indicating the position of X-ray detector data D0(view,j,i) is appended to the X-ray detector data as data of a position in the z direction of rectilinear table movement, Ztable(view), even if X-ray detector data D0(view,j,i) is acquired during acceleration or deceleration of the rotator, the direction of X-ray transmission can be accurately detected based on the geometric disposition of the focal spot and the multi-array X-ray detector.

For example, if a line partly comes out of the multi-array X-ray detector 24 in the direction of channels in the same manner as the line T0 formed by projecting the line of pixels L0 onto the surface of the multi-array X-ray detector 24 does, projection data Dr(view,x,y) forming the line is set to 0s. Moreover, if a line comes out of the multi-array X-ray detector in the z direction, missing projection data Dr(view,x,y) is extrapolated.

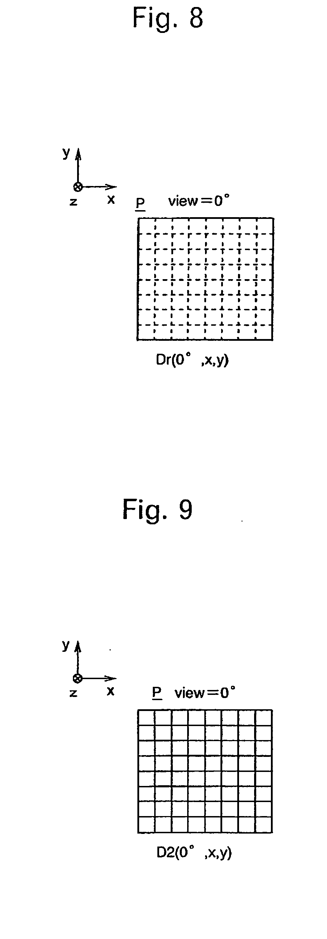

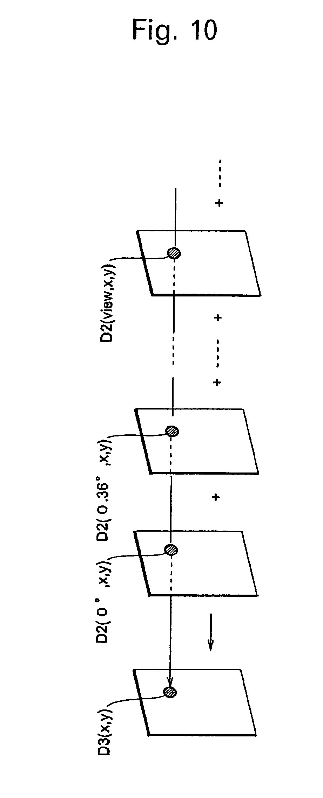

Consequently, projection data items Dr(view,x,y) associated with the pixels constituting the field of view P are sampled as shown in Fig. 8.

Referring back to Fig. 5, at step S62, the projection data items Dr(view,x,y) are multiplied by a cone-beam reconstruction weighting coefficient in order to produce projection data items D2(view,x,y) like the ones shown in Fig. 9.

Herein, the cone-beam reconstruction weighting coefficient w(i,j) will be described below. For fan-beam image reconstruction, generally, assuming that an angle at which a straight line linking the focal spot in the X-ray tube 21 that is set to a view angle view=βa and a pixel g(x,y) on the field of view P (xy plane) meets the center axis Bc of an X-ray beam is γ, and an opposed view angle is view=βb, the following relationship is established:

$$\beta b = \beta a + 180° - 2\gamma$$

Assuming that angles at which an X-ray beam passing through the pixel g(x,y) on the field of view P and the opposed X-ray beam meet the field of view P are αa and αb, projection data associated with the pixel is multiplied by cone-beam reconstruction weighting coefficients ωa and ωb dependent on the angles, and then summated in order to produce back projection pixel data D2(0,x,y).

[Formula 7]

$$D2(0,x,y) = \omega a \cdot D2(0,x,y)\_a + \omega b \cdot D2(0,x,y)\_b$$

where D2(0,x,y)_a denotes projection data produced with the X-ray tube set at a view angle βa, and D2(0,x,y)_b denotes projection data produced with the X-ray tube set at a view angle βb.

The sum of the cone-beam reconstruction weighting coefficients associated with opposite beams comes to a unity as shown below.

$$\omega a + \omega b = 1$$

When each projection data is multiplied by the cone-beam reconstruction weighting coefficients ωa and ωb, and then summated, artifacts depending on the angle of a cone beam can be reduced.

For example, the cone-beam reconstruction weighting coefficients ωa and ωb may be calculated according to expressions presented below.

Assume that a half of the angle of a fan beam is γmax.

$$ga = f(\gamma max, \alpha a, \beta a)$$

$$gb = f(\gamma max, \alpha b, \beta b)$$

$$xa = 2 \cdot ga^q / (ga^q + gb^q)$$

$$xb = 2 \cdot gb^q / (ga^q + gb^q)$$

$$\omega a = xa^2 \cdot (3-2xa)$$

$$\omega b = xb^2 \cdot (3-2xb)$$

where q equals, for example, 1.

For example, assuming that ga and gb denote functions each providing a larger max[ ] value, they are expressed as follows:

[Formula 9]

$$ga = max[0, \{(\pi/2 + \gamma max) - |\beta a|\}] \cdot |tan(\alpha a)|$$

$$gb = max[0, \{(\pi/2 + \gamma max) - |\beta b|\}] \cdot |tan(\alpha b)|$$

For fan-beam image reconstruction, the pixels constituting the field of view P are multiplied by a distance coefficient. Assuming that a distance from the focal spot in the X-ray tube 21 to a detector element that belongs to a detector array j and a channel i in the multi-array X-ray detector 24 and that produces projection data Dr is r0, and a distance from the focal spot in the X-ray tube 21 to a pixel on the field of view P associated with the projection data Dr is r1, the distance coefficient is expressed as $(r1/r0)^2$.

For parallel-rays beam image reconstruction, the pixels constituting the field of view P are multiplied by the cone-beam reconstruction weighting coefficient w(i,j) alone.

At step S63, as shown in Fig. 10, projection data items D2(view,x,y) are added pixel by pixel to back projection data items D3(x,y) that have been cleared in advance.

At step S64, steps S61 to S63 are repeated for all views needed to reconstruct a tomographic image (that is, views detected with the X-ray tube rotated 360° or an angle of 180° plus the angle of a fan beam) in order to produce back projection data items D3(x,y) as shown in Fig. 10.

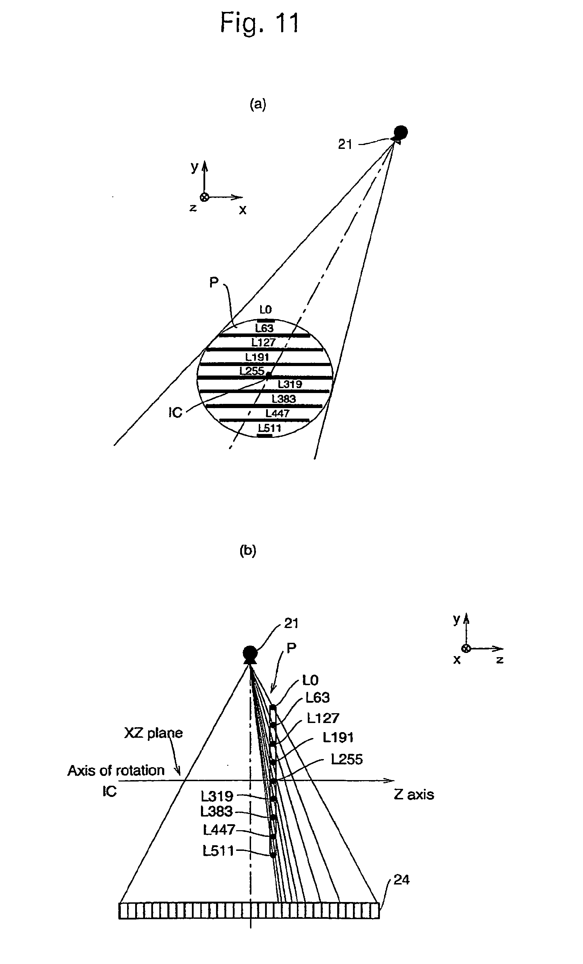

As shown in Fig. 11a and Fig. 11b, the field of view P may be a circular field.

According to an X-ray CT apparatus to which the present invention is adapted or an X-ray CT image reconstruction method in which the present invention is implemented, the X-ray CT apparatus 100 that includes a two-dimensional area X-ray detector having a matrix structure and being represented by a multi-array X-ray detector or a flat-panel X-ray detector performs a conventional (axial) scan or a cine scan. Tomographic images that enjoy a high temporal resolution or improved image quality and that are reconstructed using data items acquired synchronously with an external sync signal or a biomedical signal are three-dimensionally displayed. Otherwise, a three-dimensional tomographic image that enjoys a high temporal resolution and that is reconstructed using data items acquired synchronously with a plurality of phases of the external sync signal or biomedical signal is displayed. Otherwise, a four-dimensional tomographic image that enjoys a high temporal resolution and that is reconstructed using data items acquired synchronously with the external sync signal or biomedical signal is displayed.

As an image reconstruction method, a three-dimensional image reconstruction method based on a known Feldkamp technique may be adopted, or any other three-dimensional image reconstruction method may be adopted. Otherwise, a two-dimensional image reconstruction method may be adopted.

According to the present embodiment, especially when a conventional (axial) scan or a cine scan is performed, since a direction-of-arrays (z-direction) filter that applies different coefficients to data items detected by respective detector arrays is convoluted to the data items, a difference in image quality derived from a difference in the angle of an X-ray cone beam is adjusted to realize homogeneous image quality over the data items detected by respective detector arrays in terms of a slice thickness, artifacts, and noises. Various filtering coefficients are conceivable. In any case, the same advantage as the described one can be drawn out.

The present embodiment has been described on the assumption that the present invention is adapted to an X-ray CT apparatus for medical use. Alternatively, the present invention may be adapted to an X-ray CT apparatus for industrial use or a combination with any other modality such as an X-ray CT-PET apparatus or an X-ray CT-SPECT apparatus.

Moreover, the present embodiment has been described on the assumption that a biomedical signal is employed. In practice, if a heartbeat signal is employed, a great advantage would be expected. Moreover, a plurality of biomedical signals may be the heartbeat signal and a respiratory signal. In this case, a three-dimensional image showing the cardiac phases while being unaffected by the respiratory pulmonary motion can be displayed, or a four-dimensional image continuously showing a time-sequential change of the cardiac phases can be displayed (four-dimensional image display).

**Claims**

**1.** An X-ray CT apparatus (100) comprising:

an X-ray data acquisition device (20, 5) for rotating an X-ray generator (21) and a two-dimensional X-ray area detector (24), which is represented by a multi-array X-ray detector or a flat-panel X-ray detector and is opposed to the X-ray generator (21) in order to detect X-rays, about a center of rotation located between the X-ray generator (21) and two-dimensional X-ray area detector (24) so as to acquire X-ray projection data items of X-rays transmitted by a subject lying down between the X-ray generator (21) and two-dimensional X-ray area detector (24);
an image reconstruction device (3) for reconstructing an image using projection data items acquired by the X-ray data acquisition device (20, 5);
an image display device (6) for displaying the reconstructed tomographic image;
an external signal input device (31) for receiving an external input signal; and
a phase data input device (2) for attaining synchronization with the external sync signal, wherein:
the image reconstruction device (3) samples X-ray detector data items, which are acquired synchronously with a certain phase of the external input signal, from those acquired during cine scans, and reconstructs an image.

**2.** An X-ray CT apparatus (100) comprising:

an X-ray data acquisition device (20, 5) for rotating an X-ray generator (21) and a two-dimensional X-ray area detector (24), which is represented by a multi-array X-ray detector or a flat-panel X-ray detector and is opposed to the X-ray generator (21) in order to detect X-rays, about a center of rotation located between the X-ray generator (21) and two-dimensional X-ray area detector (24) so as to acquire X-ray projection data items of X-rays transmitted by a subject lying down between the X-ray generator (21) and two-dimensional X-ray area detector (24);

an image reconstruction device (3) for reconstructing an image using projection data items acquired by the X-ray data acquisition device (20, 5);

an image display device (6) for displaying the reconstructed tomographic image;

a biomedical signal input device (31) for receiving a biomedical signal; and

a phase data input device (2) for attaining synchronization with the biomedical signal, wherein:

the image reconstruction device (3) samples X-ray detector data items, which are acquired synchronously with a certain phase of the biomedical signal, from those acquired during cine scans, and reconstructs a three-dimensional image.

3. The X-ray CT apparatus (100) according to Claim 1 or 2, wherein the image reconstruction device (3) samples X-ray detector data items, which are acquired synchronously with a certain phase of the biomedical signal, from those acquired during cine scans so that the X-ray detector data items will correspond to those acquired during a cine scan of rotating the X-ray generator (21) by an angle of 180° plus the angle of a fan beam falling on the detector, and reconstructs an image.

4. The X-ray CT apparatus (100) according to any of Claims 1 to 3, wherein the image reconstruction device (3) samples a plurality of segments of X-ray detector data items, which is acquired synchronously with a certain phase of the biomedical signal, from those acquired during cine scans, combines or weights and summates projection data items produced from the X-ray detector data items so that view angles at which the X-ray detector data items are acquired will come to an angle of 180° plus the angle of a fan beam falling on the detector or 360°, and reconstructs an image.

5. The X-ray CT apparatus (100) according to any of Claims 1 to 4, wherein the image reconstruction device (3) samples X-ray detector data items, which are acquired synchronously with certain phases of a plurality of biomedical signals, from those acquired during cine scans so as to reconstruct an image.

6. The X-ray CT apparatus (100) according to any of Claims 1 to 5, wherein the X-ray data acquisition device (20, 5) optimizes the rotating speed of a data acquisition apparatus so that when X-ray detector data items acquired synchronously with a certain phase of a biomedical signal are sampled from those acquired during cine scans, if a temporal resolution that is a time width during which data acquisition is performed once is restricted, the X-ray detector data items can be acquired by rotating the data acquisition apparatus a small number of times.

7. The X-ray CT apparatus (100) according to any of Claims 1 to 6, wherein the image reconstruction device (3) applies a z-direction filter to projection data items during image reconstruction so that the slice thickness of a tomographic image to be reconstructed will be an arbitrary slice thickness which is in a center of image reconstruction larger than the width in a direction of arrays, that is, a z direction of the multi-array X-ray detector.

8. The X-ray CT apparatus (100) according to any of Claims 1 to 6, wherein the image reconstruction device (3) applies a z-direction filter to image data items after completion of image reconstruction so that the slice thickness of a tomographic image to be reconstructed will be an arbitrary slice thickness which is in a center of image reconstruction larger than the width in a direction of arrays, that is, a z direction of the multi-array X-ray detector.

9. The X-ray CT apparatus (100) according to any of Claims 1 to 8, wherein the image reconstruction device (3) reconstructs an image so that the slice position of a tomographic image to be reconstructed will be an arbitrary position different from a position leading to a center position of each of detector arrays, which constitute the multi-array X-ray detector, on a z axis on which a center of rotation is present.

10. The X-ray CT apparatus (100) according to any of Claims 1 to 9, wherein the image reconstruction device (3) reconstructs an image so that the inter-slice spacing of a tomographic image to be reconstructed will be an arbitrary spacing different from the spacing between adjoining ones of detector arrays, which constitute the multi-array X-ray detector, on a z axis on which a center of rotation is present.

# Fig. 1

# Fig. 2

# Fig. 3

# Fig. 4

STEP S2

# Fig. 5

STEP S6

# Fig. 6

(a)

(b)

# Fig. 7

# Fig. 8

y
z x    P    view = 0°

Dr(0°, x,y)

# Fig. 9

y
z x    P    view = 0°

D2(0°, x,y)

## Fig. 10

# Fig. 11

(a)

(b)

# Fig. 12

Helical scan

Subject

# Fig. 13

Image reconstruction performed using segment of data items acquired synchronously with biomedical signal during helical half scan (180° + angle of fan beam)

External sync signal or
biomedical signal of subject
projection data

t

one
rotation

one
rotation

one
rotation

Rotator of scanner gantry

180° + angle of fan beam

27

# Fig. 14

Image reconstruction performed using three segments corresponding to data items
acquired during helical half scan (180° + angle of fan beam)

Segment of data items
to be acquired

External sync signal or
biomedical signal of subject
projection data

one rotation    one rotation    one rotation

Rotator of scanner gantry

180° + angle of fan beam

# 図 15

Image reconstruction performed using four segments corresponding to data items
acquired during helical full scan (360°)

Segment of data items
to be acquired

External sync signal or
biomedical signal of subject
projection data

one rotation    one rotation    one rotation    one rotation

Rotator of scanner gantry

# Fig. 16

Image reconstruction performed using one segment acquired synchronously with biomedical signal during cine half scan (180° + angle of fan beam)

# Fig. 17

Image reconstruction performed using three segments of data items corresponding to
data items acquired during cine half scan (180° + angle of fan beam)

# Fig. 18

Image reconstruction performed using four segments of data items corresponding to
data items acquired during cine full scan (360°)

# Fig. 19

Method of receiving synchronizing phase of external sync signal or biomedical signal

For example, a persentage may be entered in the form of r1=40(%), r2=70(%).
Otherwise, a time may be entered in the form of r1=400(ms) or r2=700(ms).

# Fig. 20

Synchronize phase of external sync signal or biomedical signal, and switching of initiation
and termination of x-irradiation or increase and decrease of x-ray dose

# Fig. 21

Cine scan control sequence synchronized with external sync signal of biomedical signal

# Fig. 22

Reconstruction of tomographic images using data items detected by
multi-array x-ray detector 24 during cine scan

# Fig. 23

Reconstruction of successive images or time-sequential three-dimensional tomographic image
using three segments of data items that are acquired synchronously with signal and that
correspond to data items acquired during cine half scan

External sync signal or
biomedical signal of subject
projection data

Time instant $T_1$    Time instant $T_2$

$180°$ + angle of fan beam

$180°$ + angle of fan beam

| Instant $T_1$ | Instant $T_2$ |
|---|---|
| Three dimensional image showing state of subject detected at time | Time-sequential continuous display |

Time-sequential continuous display

# Fig. 24

Reconstruction of time-sequential three-dimensional tomographic image using three segments of data items
that are acquired synchronously with phases and that correspond to data items acquired dureing cine half scan

Output of x-rays

| X-irradiation | X-irradiation | X-irradiation | X-irradiation | → Time |

External sync signal
or biomedical signal

| Time instant $T_0$ | Time instant $T_1$ | Time instant $T_2$ | Time instant $T_3$ | → Time |

Phase 1
Phase 2
Phase 3
Phase 4

$180°$ + angle of fan beam

$180°$ + angle of fan beam

Time instant 2 phase 1

Time instant T1 phase 1

Time instant T1 phase 2

Time instant T1 phase 3

Time instant T1 phase 4

| Three-dimensonal image showing state of subject synchronously with phase 1 at time instant T1 | Three-dimensonal image showing state of subject synchronously with phase 2 at time instant T1 | Three-dimensonal image showing state of subject synchronously with phase 3 at time instant T1 | Three-dimensonal image showing state of subject synchronously with phase 4 at time instant T1 | Three-dimensonal image showing state of subject synchronously with phase 1 at time instant T2 |

Time-sequential continuous display

Heart

systole ————————→ diastole ————→ systole ————————→ diastole

# Fig. 25

Weighting projection each segment of data times

External sync signal or
biomedical signal of subject
projection data

Time instant T1  Time instant T2  Time instant T3  Time instant T4  t

180° + angle of fan beam

180° + angle of fan beam

Projection data items
detected at T1

Combining overlapping parts of segments
by weighting and summating them

Projection data items
detected at T2

Projection data items
detected at T3

Weighting coefficient
applied to data items
detected at T1

View

Weighting coefficient
applied to data items
detected at T2

View

Weighting coefficient
applied to data items
detected at T3

View

180° + angle of fan beam

**EP 1 736 101 A1**

European Patent Office
EUROPEAN SEARCH REPORT

Application Number
EP 06 25 3221

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DE 102 35 849 A1 (SIEMENS AG) 4 March 2004 (2004-03-04) | 1-6 | INV. A61B6/03 G06T11/00 G01N23/04 |
| Y | * page 3, paragraph 14 - page 4, paragraph 29 * * page 8, paragraphs 73,74 * * claim 16 * * page 5, paragraph 47 - page 6, paragraph 56 * | 7,8 | |
| X | EP 1 013 225 A (GENERAL ELECTRIC COMPANY) 28 June 2000 (2000-06-28) | 1,2,5 | |
| Y | * column 2, paragraph 7 - column 5, paragraph 27 * | 3,4,6 | |
| Y | US 5 751 782 A (YOSHITOME ET AL) 12 May 1998 (1998-05-12) * column 2, lines 10-56 * * column 5, line 65 - column 6, line 5 * * column 6, line 52 - column 9, line 56 * | 3,4,6 | |
| Y | EP 1 394 746 A (GE MEDICAL SYSTEMS GLOBAL TECHNOLOGY COMPANY LLC) 3 March 2004 (2004-03-03) * column 1, line 3 - column 3, line 24 * | 7,8 | TECHNICAL FIELDS SEARCHED (IPC) G01N A61B G06T |
| A | US 2004/081269 A1 (PAN TIN-SU ET AL) 29 April 2004 (2004-04-29) * page 4, paragraphs 35,36 * | 1-10 | |
| A | PATENT ABSTRACTS OF JAPAN vol. 2003, no. 12, 5 December 2003 (2003-12-05) & JP 2004 222789 A (TOSHIBA INFORMATION SYSTEMS CORP; TOSHIBA CORP), 12 August 2004 (2004-08-12) * abstract * | 1-10 | |

-/--

The present search report has been drawn up for all claims

| Place of search | Date of completion of the search | Examiner |
|---|---|---|
| Munich | 8 September 2006 | Huenges, Alexandra |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

36

**EP 1 736 101 A1**

European Patent
Office

**EUROPEAN SEARCH REPORT**

Application Number

EP 06 25 3221

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2005/069081 A1 (KOKUBUN HIROTO ET AL) 31 March 2005 (2005-03-31) * page 2, paragraph 15 * ----- | 1-10 | |
| A | PAN TINSU: "Comparison of helical and cine acquisitions for 4D-CT imaging with multislice CT" MEDICAL PHYSICS, AIP, MELVILLE, NY, US, vol. 32, no. 2, 3 February 2005 (2005-02-03), pages 627-634, XP012075260 ISSN: 0094-2405 * page 627 * * page 629, left-hand column, paragraph C * * page 630, right-hand column, last line - page 631, left-hand column, paragraph 1 * ----- | 1-10 | |
| | | | **TECHNICAL FIELDS SEARCHED (IPC)** |

The present search report has been drawn up for all claims

| Place of search | Date of completion of the search | Examiner |
|---|---|---|
| Munich | 8 September 2006 | Huenges, Alexandra |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 06 25 3221

This annex lists the patent family members relating to the patent documents cited in the above-mentioned European search report.
The members are as contained in the European Patent Office EDP file on
The European Patent Office is in no way liable for these particulars which are merely given for the purpose of information.

08-09-2006

| Patent document cited in search report | | | Publication date | Patent family member(s) | | Publication date |
|---|---|---|---|---|---|---|
| DE 10235849 | A1 | | 04-03-2004 | CN | 1480099 A | 10-03-2004 |
| | | | | JP | 2004065982 A | 04-03-2004 |
| EP 1013225 | A | | 28-06-2000 | CN | 1257693 A | 28-06-2000 |
| | | | | IL | 133325 A | 28-03-2004 |
| | | | | JP | 2000262519 A | 26-09-2000 |
| | | | | US | 6275560 B1 | 14-08-2001 |
| US 5751782 | A | | 12-05-1998 | DE | 19627166 A1 | 16-01-1997 |
| | | | | JP | 3510389 B2 | 29-03-2004 |
| | | | | JP | 9024045 A | 28-01-1997 |
| EP 1394746 | A | | 03-03-2004 | CN | 1483384 A | 24-03-2004 |
| | | | | JP | 2004073360 A | 11-03-2004 |
| | | | | US | 2004047449 A1 | 11-03-2004 |
| US 2004081269 | A1 | | 29-04-2004 | NONE | | |
| JP 2004222789 | A | | 12-08-2004 | NONE | | |
| US 2005069081 | A1 | | 31-03-2005 | CN | 1638696 A | 13-07-2005 |
| | | | | WO | 03045247 A1 | 05-06-2003 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2004275440 A